# EUROPEAN PATENT APPLICATION

(11) **EP 4 434 345 A1**
(43) Date of publication of application: **25.09.2024**
(21) Application number: 22895573.8
(22) Date of filing: 14.11.2022
(51) Int. Cl.: A01N 59/16, A01P 1/00, A61K 8/19, A61K 33/24, A61K 33/244, A61K 33/26, A61L 15/18, A61L 29/10, A61P 31/12, A61Q 15/00

(54) **ANTIVIRAL AGENT, ANTIVIRAL PRODUCT, AND ANTIVIRAL TREATMENT LIQUID**

(30) Priority: 16.11.2021 JP 2021186481
(71) Applicant: Kyodo Printing Co., Ltd., Tokyo 112-8501 (JP)
(72) Inventor: TERADA, Akira, Tokyo 112-8501 (JP); KARINO, Tomomi, Tokyo 112-8501 (JP); KOBAYASHI, Fumihito, Tokyo 112-8501 (JP); YOSHIZUMI, Wataru, Tokyo 112-8501 (JP); YAMADA, Atsushi, Yokkaichi-shi, Mie 512-0934 (JP)
(74) Representative: Ricker, Mathias
(86) International application number: PCT/JP2022/042255
(87) International publication number: WO 2023/090291

(57) **Abstract**

According to the present invention, rare-earth ferrite containing a rare-earth element, iron, and oxygen is used as an antiviral agent. Specifically, rare-earth ferrite containing a rare-earth element, iron, and oxygen is used as a main component to obtain an antiviral agent, the rare-earth element being selected from the group consisting of lanthanum, praseodymium, neodymium, and yttrium.

## Description

### FIELD

The present invention relates to an antiviral agent, an antiviral product comprising an antiviral layer comprising the antiviral agent, and an antiviral treatment liquid containing the antiviral agent.

### BACKGROUND

In recent years, from the viewpoint of hygiene, various products subjected to antibacterial treatment have been in circulation. The need for antibacterial properties has been increasing, not only for products directly used by consumers, but also for, for example, exterior and interior walls of building, building materials, air filters, and packings.

Phenol-based, organotin-based, triazine-based, sulfonylpyridine halide-based, captan-based organocopper-based, chloronaphthalene-based, and chlorophenylpyridazine-based compounds are known as chemical agents that exhibit an antibacterial effect (refer to PTL 1).

Silver ions, copper ions and zinc ions are known as ions that exhibit an antibacterial effect. Toxicity of the metal ions is utilized by supporting, for example, a metal powder of silver, copper, or Zinc, or an alloy or a compound thereof on a carrier and allowing a trace amount of metal ions to elute. PTL 2 discloses a dispersion liquid having deodorant, antibacterial, and antifungal properties, in which a carboxylate metal salt formed from a carboxyl group-containing polymer and a metal compound is dispersed.

### [CITATION LIST]

### [PATENT LITERATURE]

[PTL 1] Japanese Unexamined Patent Publication (Kokai) No. 63-17249
[PTL 2] Japanese Unexamined Patent Publication (Kokai) No. H2-288804
[PTL 3] Japanese Unexamined Patent Publication (Kokai) No. 2005-272320

### SUMMARY

### [TECHNICAL PROBLEM]

Viruses that significantly impact the human body have become a problem, such as the recent spread of the novel coronavirus. For this reason, there has been a strong demand for antiviral agents that exhibit antiviral effects.

In recent years, ferrite compounds have been proposed as anti-algae agents having a low degree of environmental contamination and excellent safety. For example, PTL 3 discloses an anti-algae additive mainly composed of an orthoferrite comprising a rare earth element selected from lanthanum (La), praseodymium (Pr), neodymium (Nd), and yttrium (Y); iron; and oxygen, an anti-algae coating material using the same, and an anti-algae product in which the coating material is applied on a substrate surface.

PTL 3 describes that presumably an anti-algae effect is associated with magnetic properties of the material, and thus orthoferrites of a rare earth oxide to Fe₂O₃ = 1:1 (molar ratio), which exhibit a small coercive force and a magnetic force close to intrinsic magnetic fields of plants, are most preferable as anti-algae additives. It should be noted that the antiviral effect of orthoferrites has not been examined in PTL 3.

In view of the above background, an object of the present invention is to provide an antiviral agent having a low degree of environmental contamination and excellent safety, an antiviral product comprising an antiviral layer comprising the antiviral agent, and an antiviral treatment liquid containing the antiviral agent.

### [SOLUTION TO PROBLEM]

The present inventors have undergone intensive studies to achieve the above object. The present inventors have discovered that a rare earth ferrite comprising a rare earth element, iron, and oxygen can be used as an antiviral (antivirus) agent having a low degree of environmental contamination and excellent safety, and have completed the present invention. Specifically, the present invention is as follows.

### <<Aspect 1>>

An antiviral agent mainly composed of a rare earth ferrite comprising: a rare earth element selected from the group consisting of lanthanum, praseodymium, neodymium, and yttrium; iron; and oxygen.

### <<Aspect 2>>

The antiviral agent according to Aspect 1, wherein the rare earth ferrite is represented by a formula (1) below:

Ln₂ₓFe₂₍₁₋ₓ₎O₃ (1)

(where in the formula (l), Ln is a rare earth element selected from the group consisting of lanthanum, praseodymium, neodymium, and yttrium, and 0 < x < 1).

### <<Aspect 3>>

The antiviral agent according to Aspect 2, where in the formula (l), x is the number of 0.45 or greater and less than 1.00.

### <<Aspect 4>>

The antiviral agent according to Aspect 2, where in the formula (l), x is the number of 0.65 or greater and 0.85 or less.

### <<Aspect 5>>

The antiviral agent according to any one of Aspects 1 to 4, wherein the rare earth element is lanthanum.

### <<Aspect 6>>

The antiviral agent according to any one of Aspects 1 to 5, which is used for an enveloped virus.

### <<Aspect 7>>

The antiviral agent according to any one of Aspects 1 to 5, which is used for a non-enveloped virus.

### <<Aspect 8>>

An antiviral product, comprising the antiviral agent according to any one of Aspects 1 to 7.

### <<Aspect 9>>

The antiviral product according to Aspect 8, comprising a substrate and an antiviral layer,
wherein the antiviral layer comprises the antiviral agent.

### <<Aspect 10>>

The antiviral product according to Aspect 9, wherein the antiviral layer further comprises a resin.

### <<Aspect 11>>

The antiviral product according to any one of Aspects 8 to 10, which is selected from the group consisting of an electrical appliance, a food hygiene product, a construction material, an interior product, a textile product, a daily necessity, a cosmetic, and a medical tool.

### <<Aspect 12>>

An antiviral treatment liquid, comprising the antiviral agent according to any one of Aspects 1 to 7.

### <<Aspect 13>>

The antiviral treatment liquid according to Aspect 12, further comprising a resin and a solvent.

### [ADVANTAGEOUS EFFECTS OF INVENTION]

According to the present invention, an antiviral agent having a low degree of environmental contamination and excellent safety is provided.

The antiviral agent of the present invention also exhibits an antiviral effect on the surroundings thereof. Specifically, the antiviral effect can spread to parts not in direct contact with the antiviral agent of the present invention. Thus, when preparing an antiviral product using the antiviral agent of the present invention, even if the antiviral agent is not present in the entirety of an article of the product, an antiviral effect can be exhibited.

The antiviral agent of the present invention has excellent dispersibility in water and organic solvents. Thus, dispersion liquids in which the antiviral agent is dispersed in various media can be prepared. Therefore, by applying a coating material comprising the antiviral agent of the present invention on an article acting as a substrate to form a layer comprising the antiviral agent, an antiviral effect can be imparted on materials on which an antiviral agent could not be applied and places having complex shapes. The dispersion liquid containing the antiviral agent of the present invention can be used as a component of antiviral sprays and the like.

By spraying or applying the dispersion liquid or gel containing the antiviral agent of the present invention as it is to the human body or an object, it is possible to immediately develop an antiviral effect.

### BRIEF DESCRIPTIONS OF DRAWING

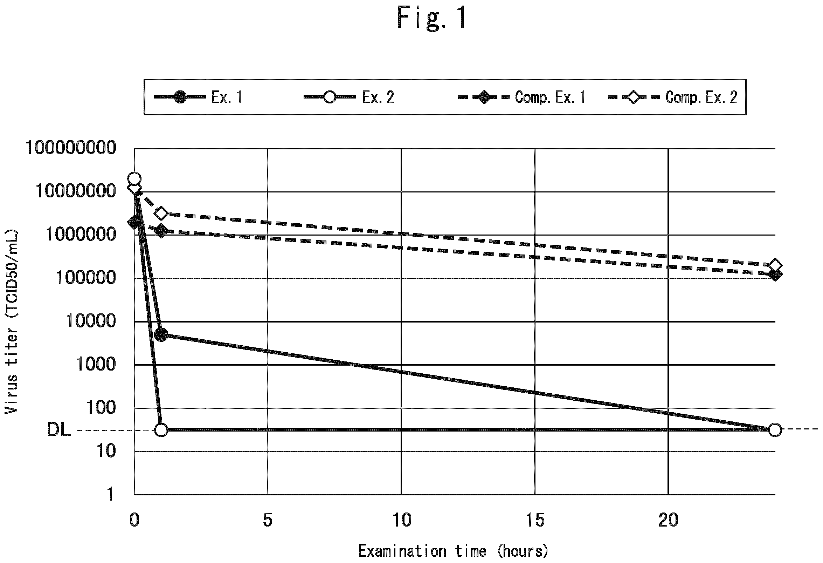

FIG. 1 is a diagram showing antiviral evaluation results in Examples and Comparative Examples.

### DESCRIPTION OF EMBODIMENTS

### <<Antiviral agent>>

The antiviral agent of the present invention is mainly composed of a rare earth ferrite comprising: a rare earth element selected from the group consisting of lanthanum, praseodymium, neodymium, and yttrium; iron; and oxygen.

The rare earth element may be a mixed rare earth mainly composed of at least one selected from the group consisting of lanthanum, praseodymium, and neodymium.

The present inventors have examined rare earth ferrites and the antiviral effect thereof in detail. As a result, the present inventors have discovered that rare earth ferrites have a high antiviral effect.

A rare earth ferrite that is a main component of the antiviral agent of the present invention may be represented by a formula (1) below:

Ln₂ₓFe₂₍₁₋ₓ₎O₃ (1)

(where in the formula (l), Ln is a rare earth element selected from the group consisting of lanthanum, praseodymium, neodymium, and yttrium, and 0 < x < 1).

The antiviral agent of the present invention may be in any form as long as it has a structure of the formula (1) above. For example, a solid solution having a uniform composition as a whole may be formed, a mixture of an LnFeOs phase and an Ln₂O₃ phase may be formed, or a mixture of a solid solution having a uniform solution, an LnFeO₃ phase and an Ln₂O₃ phase may be formed. Other phases may also be included.

The x in the formula (l) is not particularly limited as long as 0 < x < 1. For example, it may be 0.45 or greater, 0.50 or greater, 0.55 or greater, 0.60 or greater, 0.65 or greater, 0.70 or greater, or 0.75 or greater, and may be less than 1.00, 0.95 or less, 0.90 or less, 0.85 or less, 0.80 or less, 0.75 or less 0.70 or less, 0.65 or less, or 0.60 or less.

Typically, for example, the x in the formula (l) may be the number of 0.45 or greater and less than 1.00, and may further be the number of 0.65 or greater and 0.85 or less.

From the viewpoints of antiviral property and cost, the rare earth (Ln) in the formula (l) may particularly be lanthanum. Therefore, the antiviral agent of the present invention may be lanthanum ferrite.

### <Applicable virus>

Viruses to which the antiviral agent of the present invention can be applied are not particularly limited. The antiviral agent of the present invention may be used for either enveloped viruses or non-enveloped viruses and is effective for any virus.

In particular, the antiviral agent is highly effective against enveloped viruses and can be applied, for example, to the novel coronavirus (COVID-19) that occurred in 2019.

### <Production method for antiviral agent>

The production method for the antiviral agent of the present invention is not particularly limited. For example, the antibacterial agent may be produced by applying an appropriate stress to pulverize and mix a mixture containing a rare earth source and an iron source in a predetermined ratio, followed by firing the mixture. The applied stress may be, for example, frictional force, shear force, shear stress, or impact force. Examples of a method of applying such stress to the mixture of a rare earth source and an iron source include a method of wet pulverization in a ball mill.

As the rare earth source, for example, oxides of desired rare earth elements may be used, and bastnasite, monazite, and xenotime may be used.

As the rare earth element, it is preferable to use lanthanum from the viewpoints of excellent antiviral property and cost of the resulting rare earth ferrite. In particular, when La₂O₃ is used, a highly effective and relatively low-cost lanthanum ferrite can be produced.

As the iron source, oxides such as FeO, Fe₃O₄, and Fe₂O₃, oxy oxides such as FeOOH, ferrihydrite, and schwertmannite; and hydroxides such as Fe(OH)₂ and Fe(OH)₃ may be used.

Of the above, if FeOOH is used as the iron source, since the reactivity thereof is higher than Fe₂O₃, firing at a low temperature is possible, and an antiviral agent having a smaller particle size can be produced than when Fe₂O₃ is used.

When the mixture of a rare earth source and an iron source is pulverized and mixed by wet pulverization, for example, water or an alcohol may be used as a liquid medium. After the mixture of a rare earth source and an iron source is pulverized and mixed, if necessary, the liquid medium may be removed by an appropriate method such as heat drying, followed by firing.

The firing temperature is not particularly limited, and can be appropriately set. Firing may be carried out at a temperature of, for example, 500°C or higher, 600°C or higher, 700°C or higher, 800°C or higher, 900°C or higher, or 1000°C or higher and, for example, 1,300°C or lower, 1,200°C or lower, 1,100°C or lower, or 1,000°C or lower.

The firing time is also not particularly limited and can be appropriately set. For example, firing may be carried out for a time of 1 h or more, 2 h or more, 3 h or more, 4 h or more, 6 h or more, 8 h or more, 12 h or more, or 15 h or more and 72 h or less, 48 h or less, 36 h or less, 24 h or less, 18 h or less, or 15 h or less.

The ambient atmosphere during firing may be an oxidizing atmosphere, for example, firing in air.

After firing, the antiviral agent of the present invention can be obtained by pulverizing or classifying by an appropriate method, if necessary.

### <Use form of antiviral agent>

The use form of the antiviral agent of the present invention is not particularly limited, and the antibacterial agent can be used in, for example, a powder form, an impregnated form, a coating film form, or a dispersed form. The use amount thereof is not particularly limited, and can be appropriately set depending on the use form.

When the antiviral agent is used in the dispersed form, a dispersion medium is not particularly limited. The dispersion medium may be a liquid or solid. For example, when it is a liquid, it can be a dispersion liquid. When it is a solid, it can be a molded object using a resin or the like as a matrix in which the antiviral agent is dispersed.

### <<Antiviral product>>

Another aspect of the present invention is an antiviral product comprising the antiviral agent of the present invention.

Aspects of the antiviral product are not particularly limited as long as the antiviral agent of the present invention is included. For example, it may be in a solid form, liquid form, or gel-like form that is an intermediate form.

The type of antiviral product comprising the antiviral agent of the present invention is not particularly limited, and the antiviral product may be any product. For example, the antiviral product may be one selected from the group consisting of an electrical appliance, a food hygiene product, a construction material, an interior product, a textile product, a daily necessity, a cosmetic, and a medical tool. It also may be a product that has the opportunity to be touched by an unspecified number of people on public transportation or in public places such as stations and airports.

Examples of an electrical appliance include, but are not particularly limited to, a refrigerator, an air conditioner, an air purifier, a dish dryer, a microwave oven, a rice cooker, a water server, a mobile terminal, a register, a calculator, a keyboard, and a humidifier.

Examples of a food hygiene product include, but are not particularly limited to, a food storage bag, a storage container, a lunch container, a cutting board, a knife, a product processing workbench, chopsticks, a sanitary workwear, a food processing glove, a water purifier, a food packaging material, and a water bottle.

Examples of a construction material and an interior product include, but are not particularly limited to, a board flooring material, a wallpaper, a partition, a handrail, a mat, a curtain, a drainage trap, a ventilation duct, a toilet part, synthetic leather for interior use, and a decorative board.

Examples of a textile product include, but are not particularly limited to, underwear, bedding, a white coat, a mop, an insole, a hat, a towel, a mask, and an air conditioner filter.

Examples of a daily necessity include, but are not particularly limited to, a sponge cleaner, a tablecloth, a brush, an indoor deodorizer, a bathtub, and health equipment.

Examples of a cosmetic and a medical tool include, but are not particularly limited to, an antiperspirant deodorant, a cosmetic, an antiseptic, a wound dressing, a bandage, a taping tape, and a catheter.

Examples of a product that has the opportunity to be touched by an unspecified number of people in public places include, but are not particularly limited to, products located on public transportation such as trains, airplanes, buses, and taxis and products located in public places such as stations and airports. Specific examples thereof include a hanging strap, a seat, a handrail, a ticket gate, and a touch panel.

The antiviral product comprising the antiviral agent of the present invention may be an antiviral product comprising a substrate and an antiviral layer. Specifically, it comprises a substrate and an antiviral layer, and the antiviral layer is a layer comprising the antiviral agent of the present invention.

### <Substrate>

The substrate in the antiviral product may be any article to be imparted with an antiviral property. The substrate may be any article composed of any material and having any shape, wherein an antiviral property is required.

### <Antiviral layer>

The antiviral layer in the antiviral product of the present invention is not particularly limited, as long as the layer comprises the antiviral agent of the present invention. For example, the antiviral layer may be a layer prepared by blending a powder of the antiviral agent of the present invention with a resin and then carrying out extrusion coating, or may be a cured product of the antiviral treatment liquid of the present invention described below.

The antiviral layer may comprise optional components in addition to the antiviral agent of the present invention. The optional components are not particularly limited. Examples thereof include resins, binders, or additives for exhibiting a non-antiviral function.

The thickness of the antiviral layer is not particularly limited and may be, for example, about 1 µm or more and 1 mm or less.

When the antiviral layer is prepared with an antiviral treatment liquid, the application amount thereof as dry mass after solvent removal per unit area may be, for example, 5 g/m² or more, 10 g/m² or more, 15 g/m² or more, 20 g/m² or more, or 25 g/m² or more and may be, for example, 200 g/m² or less, 150 g/m² or less, 120 g/m² or less, 100 g/m² or less, 80 g/m² or less, or 70 g/m² or less.

The antiviral product may comprise a substrate and additional features other than an antiviral layer. Examples of additional features include an additional layer. The additional layer may be disposed on the outside of the substrate, between the substrate and the antiviral layer, or on the outside of the antiviral layer.

Examples of the additional layer include a primer layer formed on the substrate. By forming the antiviral layer on a primer layer, adhesion can be improved.

The deterioration of the antiviral effect due to loss or leakage of the antiviral agent from long-term use may be suppressed by laminating and covering the outside of the antiviral layer with a resin.

A hue pigment layer may be laminated on the outside of the antiviral layer to increase the degree of freedom in color design of the antiviral product. Other functional layers may be laminated thereon or combined therewith to provide a non-antiviral performance.

### <<Antiviral treatment liquid>>

Another aspect of the present invention is an antiviral treatment liquid comprising the antiviral agent of the present invention. The antiviral treatment liquid may comprise a resin and a solvent, a liquid medium such as a dispersion medium, and other components in addition to the antiviral agent of the present invention.

### <Resin>

The resin optionally contained in the antiviral treatment liquid of the present invention is not particularly limited and may be, for example, an acrylic resin, an acrylic-silicone resin, a silicone resin, an amino-alkyd resin, an epoxy resin, a phenolic resin, a urethane resin, an unsaturated polyester resin, or a fluororesin.

### <Liquid medium>

The liquid medium contained in the antiviral treatment liquid of the present invention is not particularly limited and may be selected from, for example, water, alcohols, ketones, aliphatic hydrocarbons, aromatic hydrocarbons, and esters.

### <Content of antiviral agent>

The content of the antiviral agent in the antiviral treatment liquid of the present invention as a ratio of the antiviral agent to a total of the resin and the antiviral agent in the treatment liquid when the treatment liquid contains the resin may be, for example, 1% by mass or greater, preferably 2% by mass or greater, more preferably 5% by mass or greater, even more preferably 10% by mass or greater, particularly preferably 20% by mass or greater, and especially preferably 30% by mass or greater from the viewpoint of exhibiting a high antiviral performance, and may be, for example, 80% by mass or less, preferably 70% by mass or less, more preferably 60% by mass or less, even more preferably 50% by mass or less, particularly preferably 40% by mass or less, and especially preferably 30% by mass or less or 20% by mass or less from the viewpoint of obtaining satisfactory applicability.

### <Production method for antiviral treatment liquid>

The production method for the antiviral treatment liquid of the present invention is not particularly limited. For example, the antiviral treatment liquid may be prepared by mixing a commercially available synthetic resin treatment liquid with the antiviral agent of the present invention at a predetermined ratio.

### <Application>

The antiviral treatment liquid of the present invention can be used to form the antiviral layer in the antiviral product of the present invention. Further, the antiviral treatment liquid may be used as, for example, a joint material (a filler for gaps in tiles, concrete, and bricks), a sealing material, a caulking material, an adhesive, or a spray agent. The dispersion liquid in which the antiviral agent of the present invention is dispersed in a liquid medium can be used as a component of antiviral sprays and the like. By spraying or applying the dispersion liquid as it is to the human body or an object, it is also possible to immediately develop an antiviral effect.

### EXAMPLES

### «Examples 1 and 2, Comparative Examples 1 and 2»

### <Synthesis of lanthanum ferrite>

0.35 molar parts of La₂O₃, 0.3 molar parts of FeOOH, and water were charged in a ball mill with 10 mm ϕ alumina balls as the pulverizing media and pulverized and mixed for 5 h. The resulting pulverized material was dried at 300°C for 15 h and then crushed with a rotary pulverizer. The resulting crushed material was fired and then pulverized with a hammer mill, whereby lanthanum ferrite (Formula: La_{1.4}Fe_{0.6}O₃) with La: Fe = 70:30 (molar ratio) was obtained.

### <Antiviral evaluation (measurement of decreased value of virus titer)>

Antiviral evaluation (measurement of the decreased value of virus titer) of the obtained lanthanum ferrite was conducted at Shokukanken Inc. In the antiviral evaluation, the decreased value of the virus titer of SARS-CoV-2 (novel coronavirus) over time was measured. A human-derived strain of SARS-CoV-2 (novel coronavirus) was used. Specifically, after separating and culturing Vero cells (an established cell line from the kidney epithelium of African green monkeys) from saliva, amplification of the SARS-CoV-2 gene was confirmed using real-time PCR (Official Method of Japanese Ministry of Health, Labour and Welfare). The resulting virus strain was used.

### (Preliminary test)

Before conducting a main test, 1 g and 0.1 g of the prepared lanthanum ferrite were suspended in 9 mL and 9.9 mL of physiological saline, respectively. Each was filtered through a 0.22-µm filter and serially diluted 10 times. Each diluted solution was inoculated into cultured cells and cultured for 5 days at 37°C and 5% CO₂. When the cultured cells did not show a normal shape, it was determined that the material was cytotoxic. In the main test, dilution ratios at which cytotoxicity was confirmed were excluded from the test judgment.

As a result, no cytotoxicity was observed in the 10-fold diluted solution. Therefore, the detection limit in the main test was set at 10^{1.5} TCID₅₀/mL.

### (Main test)

The liquid mixtures of Examples 1 and 2 and Comparative Examples 1 and 2 were prepared as follows, and each liquid mixture was shaken at room temperature (25°C) for a predetermined period of time.

Example 1: 9 mL of the virus liquid was added to 1g of the lanthanum ferrite.

Comparative Example 1: 9 mL of the virus liquid was added to 1 mL of physiological saline without adding the lanthanum ferrite.

Example 2: 9.9 mL of the virus liquid was added to 0.1 g of the lanthanum ferrite.

Comparative Example 2: 9.9 mL of the virus liquid was added to 0.1 mL of physiological saline without adding the lanthanum ferrite.

(1) The sensitization time was set to 1 h and 24 h for the liquid mixtures of Examples 1 and 2. The sensitization time was set to 0 h, 1 h, and 24 h for the liquid mixtures of Comparative Examples 1 and 2. The liquid mixtures were filtered through a 0.22-µm filter after each sensitization.
(2) Each filtrate was serially diluted 10 times, and 100 µL each was inoculated onto cells cultured in a 96-well plate.
(3) Each cell was cultured at 37°C under 5% CO₂ for 5 days and then observed under a microscope. The presence or absence of virus proliferation was confirmed based on the cytopathic effect (CPE) observed in cultured cells, and its concentration was calculated. The results are shown in Table 1 and FIG. 1.
(4) The decrease rate (%) in Example relative to Comparative Example was calculated for each sensitization time using the following formula. The results are shown in Table 1. Decrease rate (%) = [Concentration in Comparative Example - Concentration in Example] / [Concentration in Comparative Example] × 100

### [Table 1]

**Table 1**

| | | Sensitization time (h) | | |
|---|---|---|---|---|
| | | 0 | 1 | 24 |
| Comparative Example 1 | Virus titer (TCID₅₀/mL) | 10^{6.3} | 10^{6.1} | 10^{5.1} |
| Example 1 | Virus titer (TCID₅₀/mL) | - | <10^{1.5} | <10^{1.5} |
| | Decrease rate | - | >99% | >99% |
| Comparative Example 2 | Virus titer (TCID₅₀/mL) | 10^{7.1} | 10^{6.5} | 10^{5.3} |
| Example 2 | Virus titer (TCID₅₀/mL) | - | 10^{3.7} | <10^{1.5} |
| | Decrease rate | - | >99% | >99% |

### <Discussion>

A gradual decrease in the virus titer was observed over time for 24 h from the start of the test in Comparative Examples 1 and 2. The virus titer reached less than the detection limit one hour after the start of the test in Example 1. The decreased rate was 99% or more. The virus titer was 103.7 TCID₅₀/mL one hour after the start of the test in the Examples; however, it reached less than the detection limit 24 h later. The decreased rate was 99% or more for both 1 h and 24 h after the start of the test.

## Claims

1. An antiviral agent mainly composed of a rare earth ferrite comprising: a rare earth element selected from the group consisting of lanthanum, praseodymium, neodymium, and yttrium; iron; and oxygen.

2. The antiviral agent according to claim 1, wherein the rare earth ferrite is represented by a formula (1) below:
Ln₂ₓFe₂₍₁₋ₓ₎O₃ (1)
(where in the formula (l), Ln is a rare earth element selected from the group consisting of lanthanum, praseodymium, neodymium, and yttrium, and 0 < x < 1).

3. The antiviral agent according to claim 2, wherein in the formula (l), x is a number of 0.45 or greater and less than 1.00.

4. The antiviral agent according to claim 2, wherein in the formula (l), x is a number of 0.65 or greater and 0.85 or less.

5. The antiviral agent according to any one of claims 1 to 4, wherein the rare earth element is lanthanum.

6. The antiviral agent according to any one of claims 1 to 5, which is used for an enveloped virus.

7. The antiviral agent according to any one of claims 1 to 5, which is used for a non-enveloped virus.

8. An antiviral product, comprising the antiviral agent according to any one of claims 1 to 7.

9. The antiviral product according to claim 8, comprising a substrate and an antiviral layer,
wherein the antiviral layer comprises the antiviral agent.

10. The antiviral product according to claim 9, wherein the antiviral layer further comprises a resin.

11. The antiviral product according to any one of claims 8 to 10, which is selected from the group consisting of an electrical appliance, a food hygiene product, a construction material, an interior product, a textile product, a daily necessity, a cosmetic, and a medical tool.

12. An antiviral treatment liquid, comprising the antiviral agent according to any one of claims 1 to 7.

13. The antiviral treatment liquid according to claim 12, further comprising a resin and a solvent.
